(19)

Europäisches Patentamt
European Patent Office
Office européen des brevets

(11) **EP 2 905 544 A1**

(12) **EUROPEAN PATENT APPLICATION**

(43) Date of publication:
**12.08.2015  Bulletin 2015/33**

(51) Int Cl.:
*F24C 15/20* (2006.01)     *A61N 5/06* (2006.01)

(21) Application number: **14154414.8**

(22) Date of filing: **10.02.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Electrolux Appliances Aktiebolag**
**105 45 Stockholm (SE)**

(72) Inventors:
- **Chierchia, Lucia**
  **47122 Forli (IT)**
- **Silva, Manuel**
  **47122 Forli (IT)**

(74) Representative: **Baumgartl, Gerhard Willi**
**Electrolux Dienstleistungs GmbH**
**Group Intellectual Property**
**90327 Nürnberg (DE)**

(54) **Kitchen hood for phototherapy comprising a high illuminance light source**

(57)     A kitchen hood (1) for phototherapy, comprising at least one light source (7), wherein the at least one light source (7) is capable of emitting light with a high illuminance, preferably a maximum illuminance up to about 15,000 lux, preferably up to about 12,500 lux, more preferably up to about 10,000 lux, and wherein the at least one light source (7), more preferably, is capable of emitting a full-spectrum light. Use of kitchen hood for phototherapy. Method for treating a disease by phototherapy.

FIG 1

EP 2 905 544 A1

**Description**

The present invention relates to a kitchen hood, comprising at least one light source being capable of emitting light with a high illuminance, a light source system, use of a kitchen hood and/or a light source system for the treatment of a disease and a method for treating and/or preventing a disease, comprising a step of providing a kitchen hood, comprising at least one light source.

Seasonal affective disorder, also referred to herein as SAD, is also known as winter and/or summer depression, or seasonal depression. SAD is a mood disorder in which patients having normal mental health throughout most of the year experience depressive symptoms, such as severe mood changes, in the winter or summer, i.e. with seasonal pattern. Being a special type of affective disorder, SAD is classified according to the International Classification of Diseases ICD, particularly ICD-10, of the World Health Organization WHO, as a relapsing/recurring depressive disorder.

Besides the depressive symptoms, bad mood, reduced energy and anxiety, atypical symptoms occur, such as prolonged sleep duration, ravenous appetite for carbohydrates, especially sweets, and increase in body weight. By contrast, loss of appetite, loss of body weight and reduction of sleeping duration rather occurin non-seasonal depression.

One reason for SAD is the disturbance of the biological circadian clock. A relation of SAD and the metabolism of melatonin is assumed and thus an influence on the level of melatonin may have an anti-depressive effect. Melatonin is a product of serotonin degradation. Low levels of serotonin are, particularly in SAD, thought to be responsible for the depressive symptoms and the otherwise atypical symptoms. In winter with less sunlight occurrence SAD correlates to a lower level of serotonin and an elevated production of melatonin.

Serotonin producing cells located in the human brain release serotonin into the blood stream depended on the daytime in a circadian clock controlled manner. The circadian clock is triggered and synchronized by the light entering the eye and most likely by the light color being red and/or blue. Disturbance of serotonin release and release-rhythm due to loss or reduction of circadian clock triggers, such as reduced exposure to sunlight, may result in disorders, such as depression, more particularly SAD, and sleep disorders.

By substitution and/or replacement of circadian clock triggers, such as the provision of light by light therapy, SAD can be treated and/or prevented.

Thus, light therapy provides one kind of treatment for SAD, as well as for other disorders comprising among others skin conditions, psoriasis, vitiligo, eczema, Acne vulgaris, cancer other skin conditions, wound healing, other mood and sleep related disorders, non-seasonal depression, circadian rhythm sleep disorder, such as delayed sleep phase disorder (DSPD), shift work disorder, neonatal jaundice, and the like.

Light therapy or phototherapy consists of exposure to light having specific wavelengths and intensity using polychromatic polarized light, lasers, light-emitting diodes, fluorescent lamps, dichroic lamps or very bright, full-spectrum light, usually controlled with various devices. The light is administered for a prescribed amount of time and, in some cases, at a specific time of day.

The higher efficacy of phototherapy for e. g. SAD is now generally acknowledged by practitioners in the field.

However, the basic problem is that most time is spent indoor, for example, at indoor working places or at home. At home, light therapy can be conducted by sitting or lying in front of special lamps, such as light boxes. Also portable devices, such as flexible pads or eye surrounding elements are known.

However, in using such devices time has to be afforded especially sitting or lying in front of the light boxes. Flexible pads or eye surrounding elements restrict mobility and reduce the quality of life. Moreover, in everyday life time constraints are frequent. Still a large amount of time is spent with domestic work, such as cooking.

Although luminous structures with direct illumination in general are known in the art it is not known in the art how, phototherapy, and particularly an effective phototherapy, may be applied while carrying out domestic work, and particularly cooking. Merely, simple light-emitting diodes are used in the art insufficient for effective light therapy. It is for example known to incorporated LEDs into interior furnishings, for example, placing a diode into a recess, hole or housing, and particularly, to place the LEDs or integrate the LEDs in planar element of furnishings, for example a glass plate having transparent electrodes and having an LED placed in a recess or hole - or, alternatively, it is also known in the art to assemble LEDs on a so called PCB and to insert or integrate such PCB into such planar element. However, either the luminous structure or the used LEDs are insufficient for effective light therapy. Basically, it is preferred in the art to provide kitchen hoods comprising colored lights for chromotherapy or color therapy. In connection therewith, however it is known that chromotherapy is distinct from other types of light therapy, the latter being scientifically-accepted medical treatments for a number of conditions, as outlined above. By contrast, color therapy has been shown to have no scientifically verifiable effect.

Accordingly, the use of light therapy is preferable over the use of chromotherapy, due to the proven effect of light therapy in the treatment and/or prevention of various diseases.

It is an object of the present invention to provide light therapy within the daily environment, e. g. in the kitchen, and allowing light therapy while carrying out domestic work, especially cooking. Further it is an object of the present invention to provide sunlight-like lightning conditions during daily domestic work.

It is a still further object of the present invention to provide a method for treating and/or preventing a disease, such as SAD, during daily domestic work, especially while cooking.

The above objects of the invention are achieved by a kitchen hood according to claim 1, a light source system according to claim 13, the use of a kitchen hood and/or a light source system for the treatment of a disease according to claim 14 and a method for treating and/or preventing a disease according to claim 15.

A kitchen hood according to claim 1 comprises at least one light source, wherein the at least one light source is capable of emitting light with a high illuminance, preferably a maximum illuminance up to about 15,000 lux, preferably up to about 12,500 lux, more preferably up to about 10,000 lux, and wherein the at least one light source, more preferably, is capable of emitting a full-spectrum light.

Such kitchen hood provides a sunlight-like lightning condition, preferably, when the light source is emitting the full-spectrum light. The illuminance of up to about 15,000 lux, preferably up to about 12,500 lux, more preferably up to about 10,000 lux is provided by a light source, such as an LED and/or an RGB lamp, which is capable of emitting light with such maximum illuminance.

The term "illuminance" as used herein, preferably refers to a total luminous flux incident on a surface, per unit area. It is a measure of how much the incident light illuminates the surface, preferably wavelength-weighted by the luminosity function to correlate with human brightness perception. The SI unit of illuminance is lux (lx) or lumens per square meter (cd·sr·m-2).

A high illuminance as used herein, preferably, is an illuminance of at least about 5,000 lux, at least about 7,500, at least about 10,000 lux, at least about 12,500 lux, or at least about 15,000 lux.

It will be immediately understood that the positioning of the light-source and the inventive kitchen hood is in a position which is facing the user of said kitchen hood, wherein said position is useful for phototherapy, as the position facing the user is just above the users head or facing the users head, while the user is operating the kitchen hood and/or a stove. Thereby the user is subjected to the light emitted by the at least one light source being, preferably, capable of emitting a full-spectrum light, wherein the at least one light source is capable of emitting light with a high illuminance, preferably, a maximum illuminance up to about 15,000 lux, preferably up to about 12,500 lux, more preferably up to about 10,000 lux. A person skilled in the art will immediately acknowledge that while performing a cooking process easily is, depending on the particular receipt performed by the user, from ten to 45 minutes. Using the inventive kitchen hood for such time period, e. g. with 10,000 lux LED light for 30 minutes, this is enough to improve the mood of the user, preferably turn around bad mood of the user. Preferably, the full spectrum light has a spectrum comparable and/or imitating the spectrum of the sunlight. In an embodiment of the kitchen hood of the present invention, the kitchen hood comprises a light meter, preferably, being integrated into the hood or to be integrated into a kitchen structure, such as the stove or a kitchen plate at a defined distance. Said light meter preferably allows control of the luminance.

The term "luminance" as used herein, preferably, refers to a photometric measure of the luminous intensity per unit area of light travelling in a given direction. It more preferably describes the amount of light that passes through or is emitted from a particular area, and falls within a given solid angle. The SI unit for luminance is candela per square meter (cd/m2). It will be understood that the inventive kitchen hood, preferably, causes a light induced effect, e. g. an improvement of mood. The light induced effect is caused by illuminating or irradiating a skin area of a subject, i.e. the user that is treated with light emitted from the light-emitting light source. The luminance therefore, preferably, indicates how much luminous power will be detected by an eye looking at the surface from a particular angle of view.

The luminance as used herein, preferably, is calculated according to the following equation:

$$L_v = \frac{d^2 \Phi_v}{dA \Omega \cos \theta}$$

,

wherein

$L_v$ is the luminance in cd/m2,
$\Phi_v$ is a luminous flux or luminous power in lm,
$\theta$ is an angle between a surface normal and the specified direction,
A is an area of said surface in m2, and
$\Omega$ is a solid angle in sr.

In an advantageous embodiment of the inventive kitchen hood said at least one light source comprises at least one filter not transmitting light of a wavelength of about 400 nm or less.

In connection with the various embodiments of the inventive kitchen hood it is preferred to use a placement for the at least one light source as usually applied in the prior art and/or market, preferably a standard placement.

The term "standard placement", as used herein, preferably, refers to the placement of LEDs on a planar element, preferably a glass element, more preferably having transparent electrodes. Alternatively and/or additionally the term "standard placement" preferably, refers to LEDs being assembled on a PCB which, more preferably, is inserted onto or into a planar element. Alternatively and/or additionally, the term standard placement also, preferably, refers to a placement of LEDs placed in a recess, hole or groove, for indirect illumination. Such indirect illumination may particularly comprise the placement in a recess, hole, groove and/or housing having a concave cross-shape or profile.

In an advantageous embodiment of the inventive kitchen hood the kitchen hood and/or the at least one light source comprises at least one cover. Such cover is preferably selected from the group comprising glass cover, plastic cover or the like.

More preferably, the at least one light source is placed below said cover, i.e. the light has to pass the cover to reach the user.

It will be understood that said cover may be or may not be part of the light source. For example, a kitchen hood comprising a cover and a light source may be configured such that the light source and the cover are physically separated and/or not in contact. It may also be that such cover is in contact with or part of the at least one light source.

In an advantageous embodiment of the inventive kitchen hood and/or the at least one light source comprises at least one filter, wherein the filter is selected from the group comprising long pass filter, short pass filter and band pass filter.

In a further advantageous embodiment of the inventive kitchen hood, the kitchen hood and/or the at least one light source comprises at least one further filter, wherein the at least one further filter is selected from the group comprising long pass filter, short pass filter and band pass filter.

It will be immediately understood that different filters may thus be combined, preferably forming filter sets. For example, at least one filter being a short pass filter may be combined with at least one further filter being a long pass filter. Also one or several filters being short pass filters each having same or different wavelength-cut off values may be combined with each other and/or with one or several long pass filters having same or different wavelength-cut-on values, and vice versa. Accordingly, different settings of wavelengths may be adjusted, each setting allowing a distinct set of wavelengths to pass the filter set. In a preferred embodiment of the inventive kitchen hood filters may be exchanged and/or changed manually and/or changed automatically, preferably based on a desired program. In such case the presence of various filters allows higher flexibility and many possibilities to determine the set of wavelengths to pass the filter sets and/or the change of the set of wavelengths.

It is preferred, that the at least one cover comprises the at least one filter. For example, the filter may be a filter foil arranged on the cover.

It will be immediately understood that such filter is capable of transmitting a limited range of the spectrum and/or is capable of, or not capable of transmitting light of a certain wavelength or band of wave lengths. This allows to insure that only light of a certain spectrum will reach the user and/or, for example, UV-light will or will not reach the user.

For example, a long pass filter transmits wavelengths above a certain cut-on wavelength, wherein a short pass filter transmits wavelengths below a certain cut-off wavelength, wherein a band pass filter transmits wavelengths in a narrow range around a specified wavelength. Of course, different filters of the same or different kind may be used with the inventive kitchen hood for the same or different light source. Such filters may also be combined according to the desired wavelengths and spectrum.

In a preferred embodiment of the inventive kitchen hood, the filter is a long pass filter, not transmitting light of a wavelength of about 400 nm or less, also preferably referred to herein as a UV-filter.

It will be acknowledged that electromagnetic radiation with a wavelength shorter than that of visible light, but longer than X-rays, that is, in the range of about 400 nm and less, preferably to 10 nm, is known as ultraviolet (UV) light. It is so-named because the spectrum consists of electromagnetic waves with frequencies higher than those that humans identify as the color violet. According to the WHO UV-light has a wavelength of 400 nm or less. UV light may be further divided depended on the wave length into Ultraviolet A or UVA light having wavelength of 400 nm to 315 nm and, preferably, photon energy of 3.10 eV to 3.94 eV, Ultraviolet B or UVB light having wavelength of 315 nm to 280 nm and, preferably, photon energy of 3.94 - 4.43 eV, Ultraviolet C or UVC light having wavelength of 280 nm to 100 nm and, preferably, photon energy of 4.43 - 12.4 eV, Near Ultraviolet or NUV having wavelength of 400 nm to 300 nm and, preferably, photon energy of 3.10 - 4.13 eV, Middle Ultraviolet or MUV light having wavelength of 300 nm to 200 nm and, preferably, photon energy of 4.13 - 6.20 eV, Far Ultraviolet or FUV light having wavelength of 200 nm - 122 nm and, preferably, photon energy of 6.20 - 10.16 eV, Hydrogen Lyman-alpha or H Lyman-a light having wavelength of 122 - 121 nmand, preferably, photon energy of 10.16- 10.25 eV, Extreme Ultraviolet or UV-light having wavelength of 121 nm - 10 nmand, preferably, photon energy of 10.25 - 124 eV, and Vacuum Ultraviolet or VUV light having wavelength of 200 nm - 10 nmand, preferably, photon energy of 6.20 - 124 eV.

These frequencies are invisible to humans. However, the ultraviolet radiation exhibit an impact on the subject. UV-B light for example is improving the immune system via vitamin D, and thus is used in traditional sunlight therapy or light therapy treating tuberculosis. UV-light also may exhibit direct sterilizing effect. However, exposure of a subject's skin to UV light for a certain period of time may cause skin damage or exposure of the eyes to UV light for a certain period of time may cause damage of the eyes. Thus, for safety reasons, the kitchen hood of the invention preferably comprises a light filter or a set of light filters, not transmitting light of a certain wavelength, bandpass of wavelengths, or the like. Preferably, the kitchen hood comprises a filter not transmitting light of a wavelength of about 400 nm or less. Such filter does not allow UV light to pass the filter. The filter may be arranged such that its effect may be turned on and/or off, e. g. by the user and/or a predetermined or user determine program. However, considering non-UV phototherapy in particular, which is preferably applied in connection with the present invention, it is preferred that due to safety issues the user is not able to or at least restricted in turning off such UV-filter. In connection therewith, it will, however, be understood that for other reasons, such as sanitization or disinfection, and programs for such purposes, the filter may be arranged such that its effect may be turned on and/or off. In preferred embodiments further filters and/or set of filters are considered, not transmitting light of a certain wavelength or bands of wave length, e. g. allowing only the transmission of UVB and/or UVA light. Again for safety reasons, it is preferred that the transmission of UV light is timely controlled, such that after a predetermined time period an alarm sound warns the user and/or the filter is set on, and/or the light is turned off.

In a preferred embodiment the kitchen hood comprises a totally UV absorbent layer, e. g. an according cover and/or filter, avoiding transmission of UV light and, e. g. possible damage to the retina. It will be understood that depended on the desired effect, the transmission of UV light is allowed, the transmission of certain UV light wavelengths is allowed, and/or the transmission of UV light is not allowed. If the kitchen hood is used for prevention and/or treatment of a certain disorder, the kitchen hood can be configured accordingly, using an appropriate filter set.

Full-spectrum light as used herein, preferably, refers to light that covers the electromagnetic spectrum from infrared to ultraviolet, more preferably, full-spectrum light covers the electromagnetic spectrum from infrared to near-ultraviolet light but not ultraviolet light. In a preferred embodiment, the at least one light source is capable of emitting a full-spectrum light but no UV light. More preferably, full-spectrum light covers all wavelengths that are useful to plant or animal life, preferably, sunlight is considered full spectrum, even though the solar spectral distribution reaching earth changes with time of day, latitude, and atmospheric conditions.

In a further advantageous embodiment of the inventive kitchen hood said at least one light source comprises at least one further filter transmitting a limited range of the spectrum.

In a further advantageous embodiment of the inventive kitchen hood said at least one light source comprises at least one further filter transmitting only blue light and/or red light.

In a preferred embodiment further a cover and/or a set of covers comprises at least one filter and/or at least one further filter.

Preferably, the filter and/or cover is for changing the spectrum of the light, which, more preferably, also means for changing color of the light.

Filters may be employed which allow or not allow a light of a certain wave length to pass. For example, the color of the passing light may be restricted to blue light having a peak at, e. g. about 415 nm, or a mixed blue and red light having peaks at about 415 nm and 660 nm, or allowing blue light of wavelengths from about 400 nm to about 500 nm to transmit. In a preferred embodiment the kitchen hood of the present invention comprises at least one light source, wherein two or more light sources are considered. Thereby it is preferably considered, that for different light sources different filter combinations may be chosen or adjusted such that the same, preferably, full-spectrum light emitted by the different light sources has different colors after transmitting through the different filter or filter sets.

It will be understood that the color of a light source may be changed by adjusting the filter or filter set, being adjusted by the manufacturer or being adjustable by the user.

However, it will also be understood that in a further preferred embodiment of the kitchen hood according to the present invention the light color emitted by the kitchen hood is dependent on and/or determined by the at least one light source. In a preferred embodiment an at least one light source is an RGB lamp or a set of RGB lamps. Using RGB lamps as an at least one light source the light color may be changed by the at least one light source, preferably independent from filters or covers.

In a preferred embodiment of the kitchen hood according to the present invention the at least one light source is a multi-color LED. Such multi-color LED is capable of controlling the blending and diffusion of different colors. In a preferred embodiment the multi-color LED is selected from the group comprising di-, tri-, and tetra-chromatic LEDs. A person skilled in the art will be able to select a light source depending on the color stability, color rendering capability, and luminous efficacy.

In a preferred embodiment of the kitchen hood according to the present invention the at least one light source is an LED as usually applied in the prior art and/or market, preferably a standard LED. Such usually applied LED or LED technology is widely available in the market and advantageously allows the user to easily exchange said LED if broken or at the end of its life span.

The term "standard LED" as used herein, preferably refers to a day-light-white SMT LED.

In a preferred embodiment of the kitchen hood according to the present invention the at least one light source is a day-light-white SMT LED.

The term "SMT" as used herein, preferably refers to surface-mount technology. SMT, more preferably is a method for producing electronic circuits in which the components, such as LEDs, may be mounted or placed directly onto the surface of printed circuit boards, also referred to herein as PCBs.

Preferably, SMT is used to mount LED chips on a Printed Circuit Board.

In a preferred embodiment of the kitchen hood according to the present invention the at least one light source is an LED or a set of LEDS mounted on at least one PCB.

It is also an embodiment of the invention that different light sources not necessarily have to be turned on at the same time. Thus, allowing, for example, to switch on different light sources, preferably, having the same or different color, filter or filter settings individually and independently.

As a further preferred embodiment in addition to or as an alternative to a filter, a set of filters and/or a cover or a set of covers, an at least one light source is selected being capable of emitting colored light of a certain spectrum, preferably non-UV light.

In a further advantageous embodiment of the inventive kitchen hood said kitchen hood and/or said at least one light source is for phototherapy, preferably non-targeted phototherapy.

Light therapy or phototherapy as used herein, preferably, refers to exposure to a light spectrum, preferably, comparable or imitating daylight and/or sunlight spectrum, more preferably, using polychromatic polarized light, lasers, light-emitting diodes, fluorescent lamps, dichroic lamps or very bright, full-spectrum light. The light is administered for a certain amount of time, e. g. a prescribed cooking time, and, preferably, at a specific time of day, at a certain distance to the area of the body to be treated.

Light therapy or phototherapy as used herein preferably is for the treatment and/or prevention of disorders, such as skin disorders, sleep disorder and some psychiatric disorders. Light therapy directed at the skin is also used to treat acne vulgaris, eczema and neonatal jaundice. Light therapy which strikes the retina of the eyes is used to treat circadian rhythm disorders, such as delayed sleep phase syndrome, and can also be used to treat seasonal affective disorder, with some support for its use also with non-seasonal psychiatric disorders.

Two forms of phototherapy exist, non-targeted phototherapy, e. g. using sunlight, a light source emitting light, preferably, having sunlight spectrum or full-spectrum, a tanning booth or a light box, and targeted-phototherapy, in which light is administered to a specific, localized area of the skin. Current targeted phototherapy is administered via excimer laser, elemental gas lamp, or via LED light. Phototherapy is only administered to the affected skin, not the entire body, thus sparing healthy skin from UV rays which may lead to other health issues including skin cancer. With targeted phototherapy only being administered to the affected area of skin, more intense dosages of light can be administered, allowing skin conditions to be repaired in less time. It will be immediately understood that kitchen hood according to the present invention can be applied in such therapy, specifically, light therapy, as the at least one light source is emitting light with high a illuminance, preferably, capable of emitting a full-spectrum light. Preferably, the at least one light source is emitting light with a maximum illuminance sufficient for the treatment.

In a further advantageous embodiment of the inventive kitchen hood said kitchen hood and/or said at least one light source is for treatment and/or prevention of a disease, wherein the disease is selected preferably from the group comprising mood disorder, such as seasonal affective disorder, depression, non-seasonal depression; skin conditions and/or skin disorders, such as skin impairments, skin damages, wound healing, vitiligo, neurodermatitis, eczema, Acne, such as Acne vulgaris, or psoriasis; cancer, such as skin cancer; sleep disorders, such as circadian rhythm sleep Disorders, shift work disorder and delayed sleep phase disorder; or neonatal jaundice.

Such illness and disorders are advantageously treated and/or prevented using the kitchen hood of the present invention. Preferably, filters and filter settings, or periods of exposure to light may be set depended on the disorder or disease to be treated and/or prevented.

In a further advantageous embodiment of the inventive kitchen hood said kitchen hood comprises at least one operating element for operating the at least one light source.

Accordingly, the at least one light source may be turned on and/or off by operating said operating element. Such operating element is preferably configured as a part of the hood control. Such operating element, more preferably, is or is part of a control panel.

In a further preferred embodiment the at least one operating element allows the selection and/or adjustment and/or regulation of emitted light color, filter sets, lightning periods, programs and/or light intensity and/or brightness and/or illuminance.

The system, preferably, is to be switched ON or OFF upon user request, providing the phototherapy treatment, preferably the non-targeted phototherapy.

In a further advantageous embodiment of the inventive kitchen hood said kitchen hood comprises a memory function for storage of an operating program.

Such operating program preferably comprises information about the light settings, such as duration of light irradiation, light color, and/or filter and/or filters sets, the number, choice and timing of light sources to be turned on and/or off, light intensities emitted and the like.

Said memory function preferably is for storage of a freely creatable operating program which takes into consideration user-specific inputs and, more preferably, which can be selected by a user interface and/or the at least one operating element.

In certain embodiments the kitchen hood of the invention therefore comprises a user interface, e. g. a display.

Such operating program may be programmed and pre-stored by the manufacturer, preferably such that various light settings and durations can be realized, e. g. depending on the disorder to be treated, the color setting to be wished and/or selected by the user and/or the cooking time. Moreover, it is preferred that the user by operating the at least one operating element is able to adjust and/or modify the pre-stored programs according to the needs of the user. Said adjusted and/or modified pre-stored programs may also be stored using said memory function for repeated use thereof. It is also considered that a prescribed light therapy to prevent and/or treat a disorder of the subject may be carried out using the kitchen hood of the invention, whereby the prescribed light settings, such as light illuminance, duration, among others, may be adjusted and stored.

Various programs may be considered. Preferably, a program is considered simulating sunlight colors and/or intensities at different daytimes and/or the chance thereof depending on time and/or duration of light irradiation. Preferably, a program simulating the change in light illuminance and/or light color is considered simulating sunset or sunrise. The inventive kitchen hood preferably comprises a timer, clock and/or other time control element, such as an LED-Timer, as well as, more preferably a display and/or display function, displaying the duration of the current light exposure, remaining exposure time, title and/or other information of the selected program. Thus, the user is informed about different parameters of the program, e. g. the remaining duration. In a further embodiment of the kitchen hood of the invention the program is adjusted such that the light source is emitting light pulses at a plurality of predetermined intensities.

In accordance with the various possibilities of light settings, durations and intervals thereof, each and any light therapy, preferably non-targeted light therapy may be carried out.

In a further advantageous embodiment of the inventive kitchen hood said at least one operating element for operating the at least one light source is a remote control.

Such remote control is preferably for setting and changing parameters of the current program, but may be also used for adjusting and/or modifying, storing and/or selecting such programs. In an embodiment the remote control allows each and any adjustment to the light settings, durations and intervals, which may otherwise be set using the operating element.

In a further advantageous embodiment of the inventive kitchen hood said at least one light source is arranged such that the light emitted by the at least one light source is uniformly distributed, preferably is uniformly distributed towards the user.

Thus, the user is provided with uniform light distribution of the light emitted by the light source. Preferably, an area and/or part of the kitchen hood is arranged for said purpose, more preferably said area and/or part of the kitchen hood is an area and/or part of the kitchen hood facing the user. Is preferred that such area and/or part of the kitchen hood where the light source is integrated is configured such that the light emitted by the at least one light source is uniformly distributed, preferably is uniformly distributed towards the user. This may be realized e. g. by diffusion of the emitted light, by providing glass elements and/or reflectors and/or other appropriate measures known to a person skilled in the art.

In a further preferred embodiment the at least one light source is arranged in the bottom part of the kitchen hood, facing the kitchen hob. However, also other arrangements are possible and considered herein. In a further preferred embodiment the at least one light source is arranged in the duct of the kitchen hood. In a further preferred embodiment the at least one light source is arranged in the front panel of the kitchen hood. In a still further preferred embodiment at least one first light source is arranged in the duct of the kitchen hood and at least one second light source is arranged in the front panel of the kitchen hood.

In a further advantageous embodiment of the inventive kitchen hood said at least one light source is arranged such that the light emitted by the at least one light source radiates downwards.

Such downward radiation is arranged such that light is emitted and distributed in an angle avoiding the eyes of a user to be stroked /exposed directly, thus blinding the user is avoided and accordingly eye damages are avoided.

In an embodiment of the inventive kitchen hood said at least one light source is arranged such that the light emitted by the at least one light source radiates up-wards and/or downwards and/or sidewards.

Such up-wards and/or downwards and/or sidewards radiation is arranged such that light is emitted and distributed in an angle avoiding the eyes of a user to be stroked /exposed directly, thus blinding the user is avoided and accordingly eye damages are avoided.

It will be understood that the spatial arrangement of the kitchen hood in the kitchen is chosen depending on the cooking place and the design of the kitchen hood. In a preferred embodiment the kitchen hood is a conventional hanging kitchen hood, however other kitchen hood configurations, vertically, horizontally, fixed to a wall, horizontal or vertical telescopic or extendible kitchen hoods are considered. However, in each and any configuration it is preferred that the light is emitted and distributed in an angle avoiding the eyes of a user to be stroked /exposed directly, thus blinding the user is avoided and accordingly eye damages are avoided and aversive visual glare is minimized.

In a further advantageous embodiment of the inventive kitchen hood a distance between the at least one light source and the head of the user is at least about 20cm.

In normal operation mode an approximate constant distance of the cooking user and the kitchen hood is given. Said approximate constant distance is depending on the type of used kitchen hood, stove and the special configuration thereof. However, especially for the application of light therapy, the setting of the program parameters, e. g. illuminance and/or duration may be chosen according to said distance for higher therapy effectiveness. In an embodiment of the present kitchen hood, the distance, preferably an approximate constant distance, between kitchen hood and user is selected such that higher therapy effectiveness is ensured, more preferably said distance is from about 20cm to 60 cm, more preferably at least 20 cm, most preferably about 20 cm.

Thus, a light therapy using the kitchen hood of the invention is, for example, carried out with a light program, wherein the light source emits light having an illuminance of about 10,000 lux, a comfortable approximate constant distance is about 20 cm to 60cm and a duration of irradiation is set to 30 minutes exposure time.

In a further advantageous embodiment of the inventive kitchen hood said kitchen hood comprises a dimmer for adjusting the illuminance of the light emitted by the at least one light source.

As used herein the term dimmer preferably refers to a device used to vary the brightness of a light. It will be understood that by decreasing or increasing the RMS voltage and, hence, the mean power to the lamp, it is possible to vary the illuminance of the light output. Although variable-voltage devices are used for various purposes, the term dimmer is more preferably, a device for the control of light output from resistive incandescent, halogen, and (more recently) compact fluorescent lights (CFLs) and light-emitting diodes (LEDs).

In an embodiment the kitchen hood comprises a dimmer for adjusting the illuminance manually using the operating element and/or the remote control, or for allowing a stored program to automatically regulate light intensities depended on the chosen parameters of the program. Thus, in an embodiment also light intensities may be adjusted to the needs of the user and/or stored in a user specific or pre-determined program. Such dimmer preferably allows continuous and/or step less change of light illuminance, preferably from about 0-15,000 lux, preferably about 0-12,500 lux, more preferably from about 0 to about 10,000 lux.

The kitchen hood of the present invention therefore preferably allows the user to enjoy a sunlight-like irradiation and/or courses of light irradiation imitating the course of sunlight, e. g. a program is selectable having sun lapse, sunrise or sundown course of emitted light with regard to various parameters of the emitted light, such as spectrum, color, duration and/or illuminance. Thus the user advantageously has the ability to be subjected to sunlight-like irradiation imitating sunlight at different day times. Moreover, preferably the change of sunlight during daytime is also simulated.

The above described problems are also advantageously solved by a light source system, wherein the light source system is for being mounted to a kitchen hood, wherein the Light source system comprises at least one light source according to the present invention.

It is also an embodiment of the present invention that a light source system is provided, preferably comprising at least one light source as used in the various embodiments of the invention, particularly in the kitchen hood of the invention. Said light source system may be used for installing said light source system into kitchen hoods as well as upgrading kitchen hoods with said light source system. In accordance therewith, it is preferred that the light source system comprises mounting means for mounting and/or installing the light source system.

The above described problems are also advantageously solved by the use of kitchen hood and/or a light source system for the treatment of a disease, wherein the kitchen hood, preferably is a kitchen hood according to the present invention., wherein preferably the light source system is a light source system according to the present invention., and wherein the disease is selected preferably from the group comprising mood disorder, such as seasonal affective disorder, depression, non-seasonal depression; skin conditions and/or skin disorders, such as skin impairments, skin damages, wound healing, vitiligo, neurodermatitis, eczema, Acne, such as Acne vulgaris, or psoriasis; cancer, such as skin cancer; sleep disorders, such as circadian rhythm sleep Disorders, shift work disorder and delayed sleep phase disorder; or neonatal jaundice.

The use of the kitchen hood of the invention, for example, comprises executing a program suitable for light therapy treatment by emitting light using the kitchen hood of the invention, wherein e. g. the light source emits light having an illuminance of about 10,000 lux, a comfortable approximate constant distance is about 20cm to 60cm and a duration of irradiation is set to 30 minutes exposure time.

The above described problems are also advantageously solved by a method for treating and/or preventing a disease, comprising at least the following steps:

a) providing a kitchen hood, comprising at least one light source, wherein the at least one light source (7), preferably, is capable of emitting light with a high illuminance,

b) optionally, selecting and operating a program with parameters suitable for a light therapy treatment effective in prevention and/or treatment of the disease,

c) subjecting of a subject suffering from said disease or being at risk of suffering from said disease to light emitted by the at least one light source,

wherein the disease is selected preferably from the group comprising mood disorder, such as seasonal affective disorder, depression, non-seasonal depression; skin conditions and/or skin disorders, such as skin impairments, skin damages, wound healing, vitiligo, neurodermatitis, eczema, Acne, such as Acne vulgaris, or psoriasis; cancer, such as skin cancer; sleep disorders, such as circadian rhythm sleep Disorders, shift work disorder and delayed sleep phase disorder; or neonatal jaundice,

wherein the kitchen hood, preferably is a kitchen hood according to any one of claims 1 to 12.

The kitchen hood, preferably the kitchen hood of the present invention, is suitable to be used in a method for treating and/or preventing a disease. It is preferred that the kitchen hood of the present invention, preferably the light source according to the present invention and/or the light source system are configured and/or adjusted such that the requirements of the Directive 93/42/EEC concerning medical devices is fulfilled.

It will be understood that parameters of a program and/or the light treatment, i.e. duration, illuminance and distance to the light source are adjusted to prescribed and/or defined needs according to the particular disease the subject is at risk or suffering from.

The method for treating and/or preventing a disease according to the present invention, preferably comprising a step of providing a kitchen hood, comprising at least one light source, wherein preferably, the kitchen hood is installed and/or the at least one light source or light source system is installed such that an approximate constant distance is chosen, preferably being 20 to 60 cm. Moreover, the method preferably comprises a step of selecting and operating a program with parameters suitable for a light therapy treatment effective in prevention and/or treatment of the disease, said parameters to be adjusted more preferably being prescribed by a skilled physician, prescribing a light therapy to the subject. The method further preferably comprises subjecting of the subject suffering from said disease or being at risk of suffering from said disease to light emitted by the at least one light source, preferably according to the prescribed light therapy. The treated disease is selected preferably from the group comprising disease known to be effectively treated by light therapy. The treated disease is selected preferably from the group comprising mood disorder, such as seasonal affective disorder, depression, non-seasonal depression; skin conditions and/or skin disorders, such as skin impairments, skin damages, wound healing, vitiligo, neurodermatitis, eczema, Acne, such as Acne vulgaris, or psoriasis; cancer, such as skin cancer; sleep disorders, such as circadian rhythm sleep Disorders, shift work disorder and delayed sleep phase disorder; or neonatal jaundice.

It is immediately clear that the parameters of the light therapy and the method can be adjusted to the needs and prescribed parameters of light therapy, such as duration of exposure, distance and illuminance, according to the disease to be treated and/or prevented.

However, it will be immediately acknowledged by a person skilled in the art that, in general, and considering the major factors of clinical efficacy, ocular and dermatologic safety, and visual comfort the following is recommended:

a light source should, preferably, provide 10,000 lux at a comfortable distance to the user, whereby preferably luminance is controlled using a light meter;

the light should be projected downward toward the eyes at an angle to minimize aversive visual glare;

a light source emitting UV light, e. g. a fluorescent lamp, should have a smooth diffusing screen which filters out ultraviolet (UV) rays, as UV rays are harmful to the eyes and skin;

Blue light is speculated to be superior to red light in managing depressive symptoms which have a seasonal pattern;

as small head movements will take the eyes out of the therapeutic range of the light a relatively large scaled area of the light source and where the at least one light source is integrated, is preferred.

In accordance with the selection of parameters suitable for a light therapy treatment effective in prevention and/or treatment of a disease, it is, in general, recommended to begin light therapy with the patient receiving 10,000 lux. The dosage most often found to be effective is 5,000 lux per day, given as 2,500 lux for two hours or 10,000 lux for 30 minutes. In connection therewith, it will be acknowledged that light treatments can be administered in divided doses.

It will be acknowledged, that lower intensities are effective but take significantly more time out of each day.

The method for treating and/or preventing a disease according to the present invention, preferably the kitchen hood of the invention, for example, comprises providing a kitchen hood, comprising at least one light source, wherein the light source is at a comfortable approximate constant distance of about 20cm to 60cm, selecting and operating a program with parameters suitable for a light therapy treatment effective in prevention and/or treatment of the disease, e. g. SAD, for example an illuminance of about 10,000 lux and a duration of irradiation being 30 minutes exposure time, subjecting said subject suffering from said disease or being at risk of suffering from said disease to light emitted by the at least one light source.

It will be also immediately understood that inventive method is carried out in daily repetition, or more than once a day, according to the prescribed light therapy treatment. Executing a program suitable for light therapy treatment by emitting light using the kitchen hood of the invention, preferably comprises executing said program and subjecting the subject to said program in a daily repetition, or more than once a day, preferably according to the prescribed therapy. The method of the invention is preferably carried out once or twice a day, with e. g. 30 minutes of light exposure at 10,000 lux.

In connection therewith it is to be understood that it is the intensity of light rather than its spectrum that appears to be of critical importance in achieving an antidepressant effect.

A kitchen hood according to the present invention and the method according to the present invention can be applied in treatment of a subject suffering from a disease as described herein.

Thereby, providing an at least one light source being capable of emitting light with a high illuminance is of advantage as positive effects, such as turn of mood or antidepressant effects, can be obtained from shorter treatment sessions, e. g. 30 minutes in some patients. Studies applying light of 10,000 lux fluorescent light given for 30 minutes per day produced similar results to protocols using 2,500 lux for two hours (Magnusson et al., 1991; Terman et al., 1990a). The 10,000 lux fluorescent light box has thus become the standard in clinical practice.

A reciprocal relationship between duration and intensity of effective light treatment is assumed (J. S. Terman et al. 1990).

In a preferred embodiment of the method according to the present invention a dosage being effective in treatment of the disease is 5,000 lux per day, preferably given as 2,500 lux for two hours or 10,000 lux for 30 minutes.

The disease according to the various embodiments of the present invention is selected preferably from the group comprising mood disorder, such as seasonal affective disorder, depression, non-seasonal depression; skin conditions and/or skin disorders, such as skin impairments, skin damages, wound healing, vitiligo, neurodermatitis, eczema, Acne, such as Acne vulgaris, or psoriasis; cancer, such as skin cancer; sleep disorders, such as circadian rhythm sleep Disorders, shift work disorder and delayed sleep phase disorder; or neonatal jaundice.

More preferably, the disease according to the various embodiments of the present invention is selected from the group comprisingseasonal affective disorder, sleep disorders, depression and non-seasonal depression.

All described embodiments of the invention have the advantage that sunlight-like lightning conditions are provided during daily domestic work. Moreover, light therapy within the daily environment, e. g. in the kitchen, is made possible and allows light therapy while carrying out domestic work, especially cooking. Moreover, a method for treating and/or preventing a disease, such as SAD, during daily domestic work, especially while cooking, is provided. Furthermore a kitchen hood and a method and use thereof allows increasing life comfort by an overall increase of good mood. The invention particularly, provides a kitchen hood for the treatment of diseases, such as seasonal affective disorder, non-seasonal depressions, sleep disorders and other conditions for which the recommended medical treatment is phototherapy, with or without UV exposure. The described embodiments of the invention provide an appropriate and effective method of compensating for the lack of sunlight in the autumn and winter, especially indoor, without resorting to drugs.

The present invention will be described in further detail with reference to the drawings from which further features, embodiments and advantages may be taken, and in which

FIG 1 illustrates perspective view of a kitchen hood showing a first inventive embodiment;
FIG 2 illustrates a perspective view of a kitchen hood showing a second inventive embodiment.

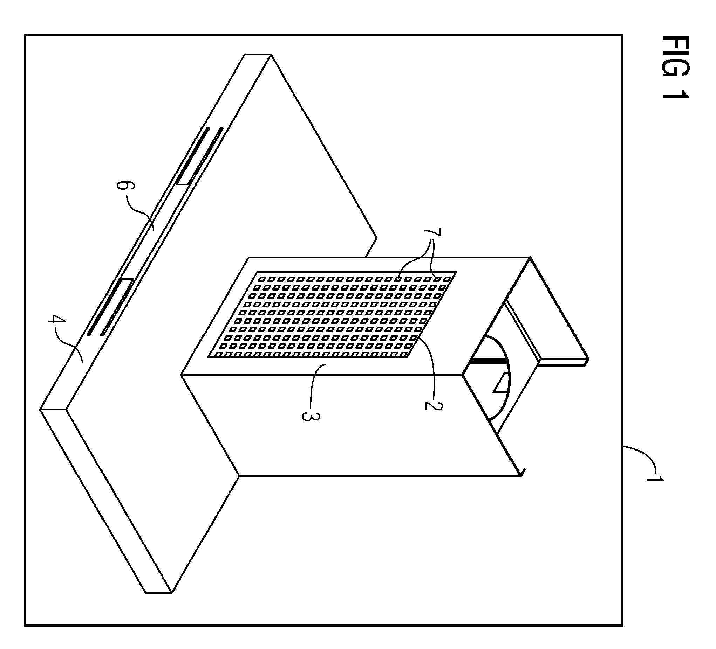

FIG 1 shows a kitchen hood (1), comprising an area (2) where at least one light source (7) is integrated. Said light source (7), here being a number of evenly distributed LEDs (7) arranged in the area (2) is capable of emitting a high illuminance, and in particular a full-spectrum light with a maximum illuminance of up to 10,000 lux. Here the LEDs are day-light-white SMT LEDs placed on a PCB. The LEDs comprise a filter (not shown here) not transmitting UV light of a wavelength of about 400 nm or less. The kitchen hood shown here is used for non-targeted phototherapy, for example of SAD. Here the full spectrum LEDs are placed in the area (2) on a PCB facing the user of the kitchen hood The kitchen hood (1) comprises a control panel (6) in its front panel (4) with which the user can control and have information regarding programs durations and settings as well as all information regarding the normal functions of the kitchen hood.

In the shown embodiment the light sources (7) and the area (2) are integrated in the duct (3) of the kitchen hood (1).

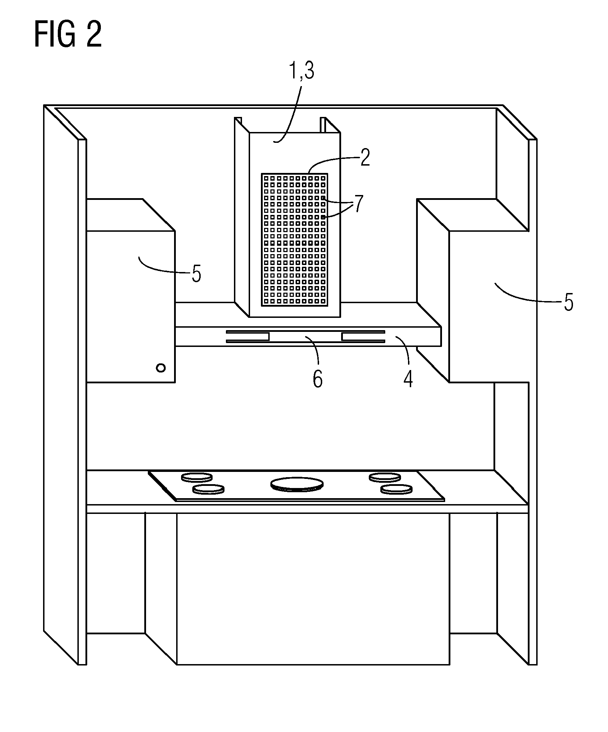

FIG 2 shows a kitchen hood (1), basically according to the kitchen hood shown in FIG 1. However, the kitchen hood (1) is installed and mounted in between kitchen cabinets (5). The kitchen hood (1) comprises a control panel (6) in its front panel (4) for operating the at least one light sources (7) and for configuring the programs stored in a memory function for storage of the operating programs. The light of the light sources (7), here being a number of evenly distributed RGB-LEDs (7) arranged in the area (2) of the duct (3) of the kitchen hood (1), is arranged in the front panel (2, 4) of the kitchen hood is arranged such that the light emitted by the at least one light source (7) is uniformly distributed, preferably is uniformly distributed towards the user, indicated by the dotted lines. Moreover the area (2) and light source (7) is arranged such that the emitted light radiates downwards. More particularly, it can be immediately understood that the positioning of the light-source and the inventive kitchen hood is in a position which is facing the user of said kitchen hood, wherein said position is useful for phototherapy, as the position facing the user is just above the users head or facing the users head, while the user is operating the kitchen hood and/or a stove.

Thereby, the user is subjected to the light emitted by the at least one light source while performing a cooking process, which easily is, depending on the particular receipt performed by the user, from ten to 45 minutes. Using the inventive kitchen hood for such time period, e. g. with 10,000 lux LED light for 30 minutes, is enough to improve the mood of the user, preferably turn around bad mood of the user.

## Example 1

A group of adult subjectssuffering from SAD, being further diagnosed by a physician, are randomly divided into one group of patients subjected to a light therapy treatment of two hours illuminance of 2,500 lux, one group subjected to treatment of 30 min with an illuminance of 10,000 lux and one control group subjected to treatment of 30 min with illuminance of 1,000 lux. The treatment is repeated daily between eight a. m. and 8. 30 a. m. for at least two weeks is carrying out the method of the present invention. According to the usual installation standards of kitchen hoods in common households, and according to an average body height of about 165 cm of female patients and of about 177 cm of male patients, a kitchen hood is provided at an approximate constant distance from about 20cm to 60 cm to the head of the subject, when the user is standing in front of the kitchen hood while cooking.

The kitchen hood provided for the method is suitable to be used in the method for treating and/or preventing SAD and the light source according to the present invention is configured and adjusted such that the requirements of the Directive 93/42/EEC concerning medical devices is fulfilled.

There are no absolute contraindications to light therapy, and there is no evidence that light therapy is associated with ocular or retinal damage, however, subjects with ocular risk factors are subjected to a baseline ophthalmological consultation prior to starting the method, and are monitored on a weekly bases.

Each patient has to fill out a questionnaire before beginning the therapy and after each week of therapy, where the mood, deviations from the prescribed protocol, and further patient-specific data, such as body weight, gender, time of being outside at daylight, illnesses and other parameters are recorded.

During light therapy treatment the luminance is controlled and recorded using a light meter.

The light therapy treatment is carried out using the arrangement shown in FIG 2. Every day the subject selects and subsequently is subjected to a program with the parameters adjusted according to the prescribed treatment of the disease to full-spectrum light emitted by the at least one light source, while performing a cooking meal. After two weeks of treatment the subject indicates to be in a better mood and having a higher live quality. One reason for SAD is the disturbance of the biological circadian clock. The repetition of the method according to the present invention with the inventive kitchen hood will show an influence on the level of melatonin and thus results in an anti-depressive effect. Melatonin is a product of serotonin degradation. After two weeks of treatment the levels of serotonin are elevated in the blood of the subject.

During the study patients are encouraged to take notes on the questionnaire concerning an optimization of the protocol.

Most patients prefer the brighter light for the shorter time because of the convenience of shortening the time required. A full 10,000 lux will also make some people's eyes uncomfortable or increase headache and eyestrain. However, 10,000 lux has been tested not causing any eye damage.

The features of the present invention disclosed in the specification, the claims, the figures and/or the Examples may both separately and in any combination thereof be material for realizing the invention in various forms thereof.

**List of reference numerals**

1    kitchen hood

2    area where the at least one light source is integrated
3    duct of kitchen hood
4    front panel of kitchen hood
5    kitchen cabinet
6    control panel
7    light source

**Claims**

1. A kitchen hood (1), comprising at least one light source (7), wherein the at least one light source (7) is capable of emitting light with a high illuminance, preferably a maximum illuminance up to about 15,000 lux, preferably up to about 12,500 lux, more preferably up to about 10,000 lux, and wherein the at least one light source (7), more preferably, is capable of emitting a full-spectrum light.

2. The kitchen hood (1) according to claim 1, wherein the kitchen hood (1) and/or the at least one light source (7) comprises at least one cover.

3. The kitchen hood (1) according to any one of claims 1 or 2, wherein the kitchen hood (1) and/or the at least one light source (7) comprises at least one filter, wherein the filter is selected from the group comprising long pass filter, short pass filter and band pass filter

4. The kitchen hood (1) according to any one of claims 1 or 3, wherein the kitchen hood (1) and/or the at least one light source (7) comprises at least one further filter, wherein the at least one further filter is selected from the group comprising long pass filter, short pass filter and band pass filter.

5. The kitchen hood (1) according to any one of claims 1 to 4, wherein the kitchen hood (1) and/or the at least one light source (7) is for phototherapy, preferably non-targeted phototherapy.

6. The kitchen hood (1) according to any one of claims 1 to 5, wherein the kitchen hood (1) and/or the at least one light source (7) is for treatment and/or prevention of a disease, wherein the disease, preferably, is selected from the group comprising mood disorder, such as seasonal affective disorder, depression, non-seasonal depression; skin conditions and/or skin disorders, such as skin impairments, skin damages, wound healing, vitiligo, neurodermatitis, eczema, Acne, such as Acne vulgaris, or psoriasis; cancer, such as skin cancer; sleep disorders, such as circadian rhythm sleep Disorders, shift work disorder and delayed sleep phase disorder; or neonatal jaundice.

7. The kitchen hood (1) according to any one of claims 1 to 6, wherein the kitchen hood (1) comprises at least one operating element (6) for operating the at least one light source (7).

8. The kitchen hood (1) according to any one of claims 1 to 7, wherein the kitchen hood (1) comprises a memory function for storage of an operating program.

9. The kitchen hood (1) according to any one of claims 1 to 8, wherein the at least one operating element (7) for operating the at least one light source (7) is a remote control.

10. The kitchen hood (1) according to any one of claims 1 to 9, wherein the at least one light source (7) is arranged such that the light emitted by the at least one light source (7) is uniformly distributed, preferably is uniformly distributed towards the user.

11. The kitchen hood (1) according to any one of claims 1 to 10, wherein the at least one light source (7) is arranged such that the light emitted by the at least one light source (7) radiates downwards.

12. The kitchen hood (1) according to any one of claims 1 to 11, wherein the kitchen hood (1) comprises a dimmer for adjusting the illuminance of the light emitted by the at least one light source (7).

13. Light source system, wherein the Light source system is for being mounted to a kitchen hood (1), wherein the light source system comprises at least one light source (7) according to any one of claims 1 to 12.

**14.** Use of kitchen hood (1) and/or a Light source system for the treatment of a disease, wherein the kitchen hood (1), preferably is a kitchen hood (1) according to any one of claims 1 to 12, wherein preferably the Light source system is a Light source system according to claim 13, and wherein the disease is selected preferably from the group comprising mood disorder, such as seasonal affective disorder, depression, non-seasonal depression; skin conditions and/or skin disorders, such as skin impairments, skin damages, wound healing, vitiligo, neurodermatitis, eczema, Acne, such as Acne vulgaris, or psoriasis; cancer, such as skin cancer; sleep disorders, such as circadian rhythm sleep Disorders, shift work disorder and delayed sleep phase disorder; or neonatal jaundice.

**15.** A method for treating and/or preventing a disease, comprising at least the following steps:

a) providing a kitchen hood (1), comprising at least one light source (7), wherein the at least one light source (7), preferably, is capable of emitting light with a high illuminance,
b) optionally, selecting and operating a program with parameters suitable for a light therapy treatment effective in prevention and/or treatment of the disease,
c) subjecting of a subject suffering from said disease or being at risk of suffering from said disease to light emitted by the at least one light source (7),

wherein the disease is selected preferably from the group comprising mood disorder, such as seasonal affective disorder, depression, non-seasonal depression; skin conditions and/or skin disorders, such as skin impairments, skin damages, wound healing, vitiligo, neurodermatitis, eczema, Acne, such as Acne vulgaris, or psoriasis; cancer, such as skin cancer; sleep disorders, such as circadian rhythm sleep Disorders, shift work disorder and delayed sleep phase disorder; or neonatal jaundice,
wherein the kitchen hood (1), preferably is a kitchen hood (1) according to any one of claims 1 to 12.

## FIG 1

# FIG 2

Europäisches
Patentamt

European
Patent Office

Office européen
des brevets

## EUROPEAN SEARCH REPORT

Application Number

EP 14 15 4414

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 428 737 A1 (BSH BOSCH SIEMENS HAUSGERAETE [DE]) 14 March 2012 (2012-03-14) | 1,2,10, 11,13 | INV. F24C15/20 A61N5/06 |
| A | * paragraphs [0030] - [0048]; figures 1-2 * | 3-9,12, 14,15 | |
| X | US 2009/080178 A1 (WILSDORF GERD [DE]) 26 March 2009 (2009-03-26) | 1,2,5-15 | |
| Y | * paragraphs [0011] - [0036]; figures 1-4 * | 3,4 | |
| X | WO 2011/139280 A1 (BROAN NU TONE LLC [US]; SINUR RICHARD R [US]; JOHNSON STEVEN A [US]; W) 10 November 2011 (2011-11-10) * paragraphs [0018] - [0046]; figures 1-3 * | 1,2,7, 10,11,13 | |
| Y | US 2005/256554 A1 (MALAK HENRYK [US]) 17 November 2005 (2005-11-17) | 3,4 | |
| A | * paragraphs [0041], [0047]; claim 15 * | 1,2,5-15 | |
| A | CN 101 934 116 A (SHANGHAI SONG S LAB TECHNOLOGY AND DEV CO LTD; SHIPENG SONG SHANGHAI S) 5 January 2011 (2011-01-05) * abstract * | 1-15 | TECHNICAL FIELDS SEARCHED (IPC) F24C A61N |
| A | US 2013/301034 A1 (OLDS JACQUELINE [US] ET AL) 14 November 2013 (2013-11-14) * paragraphs [0049], [0002], [0067], [0077], [0084], [0086] * * paragraphs [0027] - [0096]; figures 1-16 * | 1-15 | |
| A | EP 1 035 298 A1 (CHUBB CHARLES R [US]; ROTTLER LISA C [US]) 13 September 2000 (2000-09-13) * paragraphs [0029] - [0122]; figures 1-21 * | 1-15 | |

The present search report has been drawn up for all claims

| Place of search | Date of completion of the search | Examiner |
|---|---|---|
| The Hague | 5 September 2014 | Makúch, Milan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...............................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 15 4414

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2014

| Patent document cited in search report | | Publication date | Patent family member(s) | | Publication date |
|---|---|---|---|---|---|
| EP 2428737 | A1 | 14-03-2012 | DE 102010040509 | A1 | 15-03-2012 |
| | | | EP 2428737 | A1 | 14-03-2012 |
| US 2009080178 | A1 | 26-03-2009 | CN 101331361 | A | 24-12-2008 |
| | | | DE 102005060359 | A1 | 21-06-2007 |
| | | | EP 1963749 | A1 | 03-09-2008 |
| | | | US 2009080178 | A1 | 26-03-2009 |
| | | | WO 2007068584 | A1 | 21-06-2007 |
| WO 2011139280 | A1 | 10-11-2011 | NONE | | |
| US 2005256554 | A1 | 17-11-2005 | NONE | | |
| CN 101934116 | A | 05-01-2011 | CN 101934116 | A | 05-01-2011 |
| | | | WO 2012022077 | A1 | 23-02-2012 |
| US 2013301034 | A1 | 14-11-2013 | US 2013301034 | A1 | 14-11-2013 |
| | | | WO 2013106653 | A1 | 18-07-2013 |
| EP 1035298 | A1 | 13-09-2000 | AT 307264 | T | 15-11-2005 |
| | | | EP 1035298 | A1 | 13-09-2000 |
| | | | HK 1032093 | A1 | 22-12-2006 |
| | | | US 5883740 | A | 16-03-1999 |
| | | | US 5892619 | A | 06-04-1999 |
| | | | US 6017360 | A | 25-01-2000 |
| | | | US 6129438 | A | 10-10-2000 |
| | | | US 6254254 | B1 | 03-07-2001 |
| | | | US 6632002 | B1 | 14-10-2003 |
| | | | US 2002145859 | A1 | 10-10-2002 |

EPO FORM P0459

For more details about this annex : see Official Journal of the European Patent Office, No. 12/82